# NEUE EUROPÄISCHE PATENTSCHRIFT

(11) **EP 1 334 715 B2**
(45) Veröffentlichungstag und Bekanntmachung des Hinweises auf die Entscheidung über den Einspruch: **27.07.2011**
(45) Hinweis auf die Patenterteilung: 31.12.2008
(21) Anmeldenummer: 03002419.4
(22) Anmeldetag: 05.02.2003
(51) Int. Cl.: A61K 8/02, A61K 8/34, A61Q 1/14, A61Q 17/00, A61Q 19/00

(54) **Diolhaltige Reinigungszubereitungen**
Diol containing cleansing compositions
Composition nettoyante contenant un diole

(30) Priorität: 08.02.2002 DE 10205193
(43) Veröffentlichungstag der Anmeldung: 13.08.2003
(73) Patentinhaber: Beiersdorf AG, 20245 Hamburg (DE)
(72) Erfinder: Kröpke, Rainer, 22869 Schenefeld (DE); von der Fecht, Stephanie, 22869 Schenefeld (DE); Nielsen, Jens, 24558 Henstedt-Ulzburg (DE); Drucks, Anja, 22399 Hamburg (DE)

(56) Entgegenhaltungen:
- EP-A- 0 922 448
- EP-A1- 0 487 000
- EP-A1- 0 651 990
- EP-A1- 0 950 705
- EP-A2- 0 783 883
- WO-A-00/56271
- WO-A-00/56277
- WO-A-03/066011
- WO-A-03/066012
- WO-A2-01/79418
- FR-A- 2 780 283
- FR-A- 2 804 320
- FR-A1- 2 801 788
- JP-A- 11 279 023
- US-A- 5 798 111
- SPARKS KATHEEN ET AL: "MP Diol glycol: a new raw material for personal care" DRUG AND COSMETIC INDUSTRY, XX, XX, Bd. 160, Nr. 3, 1. März 1997 (1997-03-01), Seiten 30-32, XP002080226

## Beschreibung

Die vorliegende Erfindung betrifft Reinigungszubereitungen und mit dieser Reinigungszubereitung getränkte Tücher enthaltend 2-Methyl-1,3-propandiol.

Der Wunsch nach sauberer Haut ist wohl so alt wie die Menschheit, denn Schmutz, Schweiß und Reste abgestorbener Hautpartikel bieten den idealen Nährbodern für Krankheitserreger und Parasiten aller Art. Die Lust an der Körperhygiene wurde stetig verstärkt, als in den 60er Jahren des 20. Jahrhunderts neben der "klassischen" Seife auch flüssige Reinigungsmittel mit neuentwickelten synthetischen Tensiden formuliert werden konnten. Baden und Duschen sind seitdem aus unserem täglichen Leben nicht mehr wegzudenken und den Verbrauchern stehen heutzutage eine Vielzahl von Produkten für die Reinigung der verschiedenen Körperpartien zur Verfügung.

Eine besondere Gruppe an Hautreinigungsprodukten bilden dabei die Gesichtsreinigungsprodukte. Da die Gesichtshaut besonders empfindlich ist, und das Gesicht das am stärksten wahrgenommenes Körperteil und "Aushängeschild" des Menschen ist, werden für die Gesichtsreinigung besonders milde und die Haut nicht reizende Produkte eingesetzt.

Im Bereich der dekorativen Kosmetik finden eine Vielzahl von unterschiedlichen Stoffen Anwendung. Als Farbstoffe werden neben anorganischen Pigmenten wie Silikaten [Magnesiumsilikat (Talkum), Aluminiumsilikat (Kaolin)] und Metalloxiden (Chrom-, Eisen-, Mangan-, Titan- und Zinkoxiden) organische Farbpigmente eingesetzt. Als Bindemittel finden unter anderem Stearinsäureester, Lanolinalkohol und -acetat Verwendung. In vielen Formulierungen werden Wachse wie Bienenwachs oder Carnaubawachs und Öle wie Paraffinöle, Silikonöle 6der Ricinusöl eingesetzt. Des weiteren können dekorative Kosmetika Konservierungsstoffe, Antioxidantien, Verdickungsmittel und andere Zusätze enthalten.

Um diese Vielzahl völlig unterschiedlicher Stoffe von der Haut zu entfernen, bedarf es entsprechender kosmetischer Reinigungsmittel. Sie müssen unpolare Verbindungen wie Wachse, Öle und Silikonverbindungen lösen und gleichzeitig die schwerlöslichen Pigmente wie Talkum oder Titandioxid aufnehmen. Dies gilt insbesondere für Wimpemtusche, Mascara, Lidschatten und Kajalstifte. Andererseits müssen sie so hautverträglich wie möglich sein, um bei den Anwendern keine Hautrötungen oder Schleimhautirritationen auszulösen.

Eine besondere Ausführungsform kosmetischer Reinigungsmittel stellen die Tücher dar. Diese können mit einer Reinigungszubereitung getränkt sein, welche die mechanische Entfernung des Schmutzes von der Haut unterstützt. Kommerziell erhältliche, mit Reinigungsmitteln getränkte Tücher haben darüber hinaus den Vorteil, dass in ihnen die Reinigungszubereitung bereits in der richtigen Menge vorgegeben ist. Außerdem vermeiden sie die Nachteile von in Flaschen aufbewahrten Reinigungsmitteln, deren Verpackung zerbrechen und deren Inhalt "auslaufen" kann.

Die Nachteile des Standes der Technik liegen in der Reinigungsleistung der Reinigungszubereitungen, welche häufig zu wünschen übrig läßt. Besonders pigmenthaltige (dekorative) Kosmetika sind schwer von der Haut zu entfernen. Auch lassen diese Zubereitungen in der Regel ein unangenehmes Gefühl auf der Haut zurück welches unter anderem durch die relativ großen Mengen an Tensiden in den Zubereitungen verursacht wird. Nicht zuletzt werden Reinigungszubereitungen und mit Reinigungszubereitungen getränkte Tücher mit relativ großen Mengen an Konservierungsstoffen stabil und haltbar gemacht. Diese Stoffe finden jedoch beim Verbraucher nur wenig Akzeptanz.

Es war daher die Aufgabe der vorliegenden Erfindung, die Mängel des Standes der Technik zu beseitigen und kosmetische Zubereitungen zur Reinigung der Haut zu entwickeln, deren Reinigungskraft und sensorischen Eigenschaften verbessert und deren Gehalt an Koservierungsstoffen deutlich reduziert ist.

Überraschend gelöst wird die Aufgabe durch kosmetische Zubereitungen zur Reinigung der Haut enthaltend
a) 1-8 Gewichts-% 2-Methyl-1,3-propandiol,
b) eine Ölkomponente gewählt aus den Verbindungen Octylpalmitat, Octylcocoat,
   Octylisostearat, Octyldodeceylmyristat, Cetearylisononanoat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat.

Zwar beschreiben die WO 00/56277 und die WO 00/56271 Tücher die mit u.a. 2-Methyl-1,3-propandiol imprägniert sein können, doch enthalten diese Imprägnierungen astringente Salze (WO 00/56277) beziehungsweise α-Hydroxycarbonsäuren (WO 00/56271) als zwingende Bestandteile und konnten nicht den Weg zu den erfindungsgemäßen Zubereitungen weisen.

Darüber hinaus waren dem Fachmann die US 5798111, EP 0922448, FR 2780283, FR 2804320 sowie
K. Sparks et al. "MP Diol glycol: a new raw material for personal care" Drug and cosmetic Industry, Bd.160, Nr 3, 1. März 1997, Seiten 30-32.
P. Piep, Das große Rezeptbuch der Haut- und Körperpflegemittel, Dr. Alfred Hüthig Verlag GmbH, 3. Auflage, 1996, Seiten 516-527,
H. Janistyn, Taschenbuch der moderenen Parfümerie und Kosmetik, Wissenschaftliche Verlagsgesellschaft Stuttgart, 4. Auflage, 1974, Seiten 539-540,
bekannt doch konnten diese Schriften nicht den Weg zur vorliegenden Erfindung weisen, da die hier beschriebenen Zubereitungen entweder kein 2-Methyl-1,3-propandiol oder keine der erfindungsgemäßen Ölkomponenten enthalten.

Die erfindungsgemäßen kosmetischen Zubereitungen enthalten 2-Methyl-1,3-propandiol in einer Konzentration von 1 bis 8 Gewichts-%, jeweils bezogen auf das Gesamtgewicht der Zubereitung.

Die erfindungsgemäßen kosmetischen Zubereitungen können erfindungsgemäß vorteilhaft ein oder mehrere waschaktive anionische, kationische, amphotere und/oder nicht-ionische Tenside enthalten. Es ist besonders vorteilhaft das oder die erfindungsgemäßen waschaktiven Tenside aus der Gruppe der Tenside zu wählen, welche einen HLB-Wert von mehr als 25 haben, ganz besonders vorteilhaft sind solche, welchen einen HLB-Wert von mehr als 35 haben.

Besonders vorteilhafte waschaktive anionische Tenside im Sinne der vorliegenden Erfindung sind
Acylaminosäuren und deren Salze, wie
■ Acylglutamate, insbesondere Natriumacylglutamat
■ Sarcosinate, beispielsweise Myristoyl Sarcosin, TEA-lauroyl Sarcosinat, Natriumlauroylsarcosinat und Natriumcocoylsarkosinat,
Sulfonsäuren und deren Salze, wie
■ Acyl-isethionate, z.B. Natrium-/ Ammoniumcocoyl-isethionat,
■ Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat
sowie Schwefelsäureester, wie
■ Alkylethersulfat, beispielsweise Natrium-, Ammonium-, Magnesium-, MIPA-, TIPA- Laurethsulfat, Natriummyrethsulfat und Natrium C₁₂₋₁₃ Parethsulfat,
■ Alkylsulfate, beispielsweise Natrium-, Ammonium- und TEA- Laurylsulfat.

Besonders vorteilhafte waschaktive kationische Tenside im Sinne der vorliegenden Erfindung sind quarternäre Tenside. Quaternäre Tenside enthalten mindestens ein N-Atom, das mit 4 Alkyl- oder Arygruppen kovalent verbunden ist. Vorteilhaft sind Benzalkoniumchlorid, Alkylbetain, Alkylamidopropylbetain und Alkyl-amidopropylhydroxysultain.

Besonders vorteilhafte waschaktive amphotere Tenside im Sinne der vorliegenden Erfindung sind
■ Acyl-/dialkylethylendiamine, beispielsweise Natnumacylamphoacetat, Dinatriumacylamphodipropionat, Dinatriumalkylamphodiacetat, Natriumacylamphohydroxypropylsulfönat, Dinatriumacylamphodiacetat und Natriumacylamphopropionat,

Besonders vorteilhafte waschaktive nicht-ionische Tenside im Sinne der vorliegenden Erfindung sind
■ Alkanolamide, wie Cocamide MEA/ DEA/ MIPA,
■ Ester, die durch Veresterung von Carbonsäuren mit Ethylenoxid, Glycerin, Sorbitan oder anderen Alkoholen entstehen,
■ Ether, beispielsweise ethoxylierte Alkohole, ethoxyliertes Lanolin, ethoxylierte Polysiloxane, propoxylierte POE Ether und Alkylpolyglycoside wie Laurylglucosid, Decylglycosid und Cocoglycosid.

Weitere vorteilhafte anionische Tenside sind
■ Taurate, beispielsweise Natriumlauroyltaurat und Natriummethylcocoyltaurat,
■ Ether-Carbonsäuren, beispielsweise Natriumlaureth-13 Carboxylat und Natrium PEG-6 Cocamide Carboxylat,
■ Phosphorsäureester und Salze, wie beispielsweise DEA-Oleth-10 Phosphat und Dilaureth-4 Phosphat,
■ Alkylsulfonate, beispielsweise Natriumcocosmonoglyceridsulfat, Natrium C₁₂₋₁₄ Olefin-sulfonat, Natnumlaurylsulfoacetat und Magnesium PEG-3 Cocamidsulfat
■ Natrium-, Kalium-, Triethanolammoniumsalze von Fettsäuren.

Weitere vorteilhafte amphotere Tenside sind
■ N-Alkylaminosäuren, beispielsweise Aminopropylalkylglutamid, Alkylaminopropionsäure, Natriumalkylimidodipropionat und Lauroamphocarboxyglycinat.

Weitere vorteilhafte nicht-ionische Tenside sind Alkohole.

Weitere geeignete anionische Tenside im Sinne der vorliegenden Erfindung sind ferner
■ Acylglutamate wie Di-TEA-palmitoylaspartat und Natrium Caprylic/ Capric Glutamat,
■ Acylpeptide, beispielsweise Palmitoyl hydrolysiertes Milchprotein, Natrium Cocoyl hydrolysiertes Soja Protein und Natrium-/ Kalium Cocoyl hydrolysiertes Kollagen
sowie Carbonsäuren, insbesondere Fettsäuren mit C-8 bis C-22 Kohlenstoffatomen, und Derivate, wie
■ beispielsweise Laurinsäure, Aluminiumstearat, Magnesiumalkanolat und Zinkundecylenat,
■ Ester-Carbonsäuren, beispielsweise Calciumstearoyllactylat, Laureth-6 Citrat und Natrium PEG-4 Lauramidcarboxylat,
■ Alkylarylsulfonate.

Weitere geeignete kationische Tenside im Sinne der vorliegenden Erfindung sind ferner
■ Alkylamine,
■ Alkylimidazole und
■ ethoxylierte Amine.

Weitere geeignete nicht-ionische Tenside im Sinne der vorliegenden Erfindung sind ferner Aminoxide, wie Cocoamidopropylaminoxid.

Es ist vorteilhaft im Sinn der vorliegenden Erfindung, wenn der Gehalt an einem oder mehreren waschaktiven Tensiden in der kosmetischen Zubereitung aus dem Bereich von 0,1 bis 25 Gew.-%, ganz besonders vorteilhaft von 1 bis 10 Gew.-% gewählt wird, jeweils bezogen auf das Gesamtgewicht der Zubereitungen.

Erfindungsgemäß vorteilhaft können die erfindungsgemäßen Zubereitungen Polysorbate enthalten. Polysorbate stellen eine Verbindungsklasse dar, die sich vom Sorbitan, einem aus Sorbit durch Abspaltung zweier Äquivalente Wasser gewonnenem Furanderivat, ableiteten. Die Hydroxylgruppen des Sorbitans sind mit Polyethylenglykolen verethert, deren Enden mit Fettsäuren verestert sein können. Sie lassen sich allgemein durch die Formel 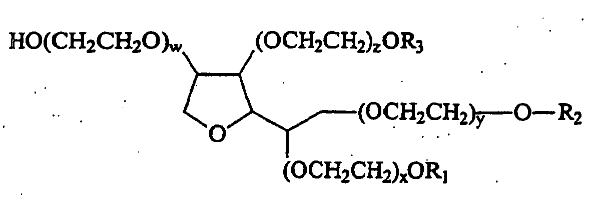 R₁, R₂, R₃ = H, Fettsäurerest
darstellen.

Im Sinne der Erfindung vorteilhafte Polysorbate sind beispielsweise das
- Polyoxyethylen(20)sorbitanmonolaurat (Tween 20, CAS-Nr.9005-64-5)
- Polyoxyethylen(4)sorbitanmonolaurat (Tween 21, CAS-Nr.9095-64-5)
- Polyoxyethylen(4)sorbitanmonostearat (Tween 61, CAS-Nr. 9005-67-8)
- Polyoxyethylen(20)sorbitantristearat (Tween 65, CAS-Nr. 9005-71-4)
- Polyoxyethylen(20)sorbitanmonooleat (Tween 80, CAS-Nr. 9005-65-6)
- Polyoxyethylen(5)sorbitanmonooleat (Tween, 81, CAS-Nr. 9005-65-5)
- Polyoxyethylen(20)sorbitantrioleat (Tween 85, CAS-Nr. 9005-70-3).

Diese werden erfindungsgemäß vorteilhaft in einer Konzentration von 0,1 bis 5 Gewichts-% und insbesondere in einer Konzentration von 1,5 bis 2,5 Gewichts-%, bezogen auf das Gesamtgewicht der Zubereitung einzeln oder als Mischung mehrer Polysorbate, eingesetzt.

Ferner ist es erfindungsgemäß von Vorteil, wenn in den erfindungsgemäßen Zubereitungen Verdicker eingesetzt werden. Diese können beispielsweise vorteilhaft aus der Gruppe der Gummen gewählt werden.

Zu den Gummen zählt man Pflanzen- oder Baumsäfte, die an der Luft erhärten und Harze bilden oder Extrakte aus Wasserpflanzen. Aus dieser Gruppe können vorteilhaft im Sinne der vorliegenden Erfindung gewählt werden beispielsweise Gummi Arabicum, Johannisbrotmehl, Tragacanth, Karaya, Guar Gummi, Pektin, Gellan Gummi, Carrageen, Agar, Algine, Chondrus, Xanthan Gummi.

Weiterhin vorteilhaft ist die Verwendung von derivatisierten Gummen wie z.B. Hydroxypropyl Guar (Jaguar® HP 8).

Unter den Polysacchariden und -derivaten befinden sich z.B. Hyaluronsäure, Chitin und Chitosan, Chondroitinsulfate, Stärke und Stärkederivate als erfindungsgemäß vorteilhafte Verdicker.

Unter den Cellulosederivaten befinden sich z.B. Methylcellulose, Carboxymethylcellulose, Hydroxyethylcellulose, Hydroxypropylmethylcellulose als erfindungsgemäß vorteilhafte Verdicker.

Unter den Schichtsilikaten befinden sich natürlich vorkommende und synthetische Tonerden wie z.B. Montmorillonit, Bentonit, Hektont, Laponit,. Magnesiumaluminiumsilikate wie Veegum®. Diese können als solche oder in modifizierter Form als Verdicker verwendet werden wie z.B. Stearylalkonium Hektorite.

Weiterhin können vorteilhaft auch Kieselsäuregele verwendet werden.

Unter den Polyacrylaten befinden sich z.B. Carbopol Typen der Firma NOVEON (Carbopol 980, 981, 1382, 5984, 2984, ETD 2001, ETD 2020, ETD 2050 oder Pemulen TRI & TR2).

Unter den Polymeren befinden sich z.B. Polyacrylamide (Seppigel 305), Polyvinylalkohole, PVP, PVP / VA Copolymere, Polyglycole.

Vorteilhaft, wenn auch nicht zwingend, können die erfindungsgemäßen Zubereitungen Konservierungsmittel enthalten
Vorteilhafte Konservierungsmittel im Sinne der vorliegenden Erfindung sind beispielsweise Formaldehydabspalter (wie z. B. DMDM Hydantom, welches beispielsweise unter der Handelsbezeichnung Glydant ™ von der Fa. Lonza erhältlich ist), lodopropylbutylcarbamate (z. B. die unter den Handelsbezeichnungen Glycacil-L, Glycacil-S von der Fa. Lonza erhältlichen und/oder Dekaben LMB von Jan Dekker), Parabene (d.h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfaßt das Konservierungssystem erfindungsgemäß ferner vorteilhaft auch Konservierungshelfer, wie beispielsweise Octoxyglycerin, Glycine Soja etc.

Weiterhin können die erfindungsgemäßen Zubereitungen Copolymere enthalten. Dabei sind die Styren/Acrylat Copolymere erfindungsgemäß von Vorteil, im Besonderen in Konzentrationen von 0,1 bis 5 Gewichts-%.

Des Weiteren können in die erfindungsgemäßen Zubereitungen die in der Kosmetik üblichen Hilfs- und Zusatzstoffe eingearbeitet werden, beispielsweise
- Antioxidantien
- Parfüm
- Farbstoffe
- Puffersysteme
- Glyceride
- Feuchthaltemittel (Moisturizer)

Polare Ölkomponenten können im Sinne der vorliegenden Erfindung gewählt werden aus Octylpalmitat, Octylcocoat, Octylisostearat, Octyldodeceylmyristat, Cetearylisononanoat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat.

Vorteilhaft kann die Lipid phase ferner einen Gehalt an, cyclischen oder linearen Silikonölen aufweisen, wobei allerdings außer dem Silikonöl oder den Silikonölen ein zusätzlicher Gehalt an anderen Ölphasenkompönenten zu verwenden ist.

Silikonöle sind hochmolekulare synthetische polymere Verbindungen, in denen Silicium-Atome über Sauerstoff-Atome ketten- und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind. Systematisch werden die Silikonöle als Polyorganosiloxane bezeichnet. Die methylsubstituierten Polyorganosiloxane, welche die mengenmäßig bedeutendsten Verbindungen dieser Gruppe darstellen und sich durch die folgende Strukturformel auszeichnen 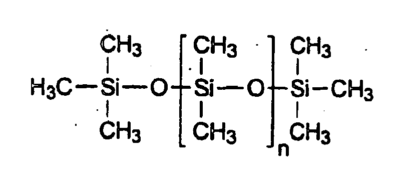 werden auch als Polydimethylsiloxan bzw. Dimethicon (INCI) bezeichnet. Dimethicone gibt es in verschiedenen Kettenlängen bzw. mit verschiedenen Molekulargewichten.

Besonders vorteilhafte Polyorganosiloxane im Sinne der vorliegenden Erfindung sind beispielsweise Dimethylpolysiloxane [Poly(dimethylsiloxan)], welche beispielsweise unter den Handelsbezeichnungen Abil 10 bis 10 000 bei Th. Goldschmidt erhältlich sind. Ferner vorteilhaft sind Phenylmethylpolysiloxane (INCI: Phenyl Dimethicone, Phenyl Trimethicone), cyclische Silikone (Octamethylcyclotetrasiloxan bzw. Decamethylcyclopentasiloxan), welche nach INCl auch als Cyclomethicone bezeichnet werden, aminomodifizierte Silikone (INCI: Amodimethicöne) und Silikonwachse, z. B. Polysiloxan-Polyalkylen-Copolymere (INCI: Stearyl Dimethicone und Cetyl Dimethicone) und Dialkoxydimethylpolysiloxane (Stearoxy Dimethicone und Behenoxy Stearyl Dimethicone), welche als verschiedene Abil-Wax-Typen bei Th. Goldschmidt erhältlich sind. Aber auch andere Sihkonöle sind vorteilhaft im Sinne der vorliegenden Erfindung zu verwenden, beispielsweise Cetyldimethicon, Hexamethylcyclotrisiloxan, Polydimethylsiloxan, Poly(methylphenylsiloxan).Erfindungsgemäß vorteilhaft kann der Lipidanteil 0,1 bis 20 Gewichts-% und besonders bevorzugt 10 bis 20 Gewichts-%, bezogen auf das Gesamtgewicht der Zubereitung betragen.

Es kann weiterhin erfindungsgemäß von Vorteil sein, der erfindungsgemäßen Zubereitung Farbpartikel, z.B. Wirkstoffkügelchen Zuzusetzen. Durch eingearbeitete Farbpigmente, Gasbläschen und dergleichen, oder aber auch durch größere Objekte und Farbschlieren lassen sich die erfindungsgemäßen Hautreinigungszubereitungen in erfindungsgemäß vorteilhafter Form optisch interessant gestalten.

Vorteilhaft im Sinne der Erfindung ist ferner die Tränkung von unlöslichen Substraten wie Tüchern, Watte und anderen mit der erfindungsgemäßen Zubereitung.

Eine besonders bevorzugte Ausführungsform stellen Tücher dar, die mit der erfindungsgemäßen Zubereitung getränkt sind.

Die erfindungsgemäßen kosmetischen oder dermatologischen Tücher können söwoh aus wasserlöslichen (z. B. wie Toilettenpapier) als auch aus wasserunlöslichen Materialien bestehen. Ferner können die Tücher glatt oder auch oberflächenstrukturiert sein. Erfindungsgemäß besonders bevorzugt sind oberflächenstrukturierte Tücher.

Erfindungsgemäß bevorzugt werden "trockene" Tücher eingesetzt, welche aus Vlies bestehen, insbesondere aus wasserstrahlverfestigten und/oder wasserstrahlgeprägten Vlies.
Derartige erfindungsgemäße Vliese können Makroprägungen jeden gewünschten Musters aufweisen.

Die Dicke des Vlieses beträgt vorzugsweise 0,4 mm bis 2 mm, insbesondere 0,6 mm bis 0,9 mm.

Als Ausgangsmaterialien für den Vliesstoff des Tuches können generell alle organischen und anorganischen Faserstoffe auf natürlicher und synthetischer Basis verwendet werden. Beispielhaft seien Viskose, Baumwolle, Zellulose, Jute, Hanf, Sisal, Seide, Wolle, Polypropylen, Polyester, Polyethylenterephthalat (PET), Aramid, Nylon, Polyvinylderivate, Polyurethane, Polylactid, Polyhydroxyalkanoat, Celluloseester und/oder Polyethylen sowie auch mineralische Fasern wie Glasfasern oder Kohlenstoffasern angeführt. Die vorliegende Erfindung ist aber nicht auf die genannten Materialien beschränkt, sondern es können eine Vielzahl weiterer Fasern zur Vliesbildung eingesetzt werden. Es ist insbesondere vorteilhaft im Sinne der vorliegenden Erfindung, wenn die eingesetzten Fasern nicht wasserlöslich sind.

In einer besonders vorteilhaften Ausführungsform des Vlieses bestehen die Fasern aus einer Mischung aus 70 % Viskose und 30 % PET.

Besonders vorteilhaft sind auch Fasern aus hochfesten Polyme ren wie Polyamid, Polyester und/oder hochgerecktem Polyethylen.

Darüber hinaus können die Fasern auch eingefärbt sein, um die optische Attraktivität des Vlieses betonen und/oder erhöhen zu können. Die Fasern können zusätzlich UV-Stabilsatoren und/oder Konservierungsmittel enthalten.

Es hat sich als vorteilhaft herausgestellt für das Tuch, wenn dieses ein Gewicht von 35 bis 120 g/m², vorzugsweise von 40 bis 60 g/m², hat (gemessen bei 20 °C ± 2 °C und bei einer Feuchtigkeit der Raumluft von 65 % ± 5 % für 24 Stunden).

Die zur Bildung des Tuches eingesetzten Fasern weisen vorzugsweise eine Wasseraufnahmerate von mehr als 60 mm/[10 min] (gemessen mit dem EDANA Test 10.1-72), insbesondere mehr als 80 mm/[10 min] auf.

Fernen weisen die zur Bildung des Tuches eingesetzten Fasern vorzugsweise ein Wasseraufnahmevermögen von mehr als 5 g/g (gemessen mit dem EDANA Test 10.1-72), insbesondere mehr als 8 g/g auf.

Vorteilhafte Tücher im Sinne der vorliegenden Erfindung haben eine Reißkraft von insbesondere

| | | [N/50mm] |
|---|---|---|
| im trockenen Zustand | Maschinenrichtung | >60, vorzugsweise >80 |
| | Querrichtung | >20, vorzugsweise >30 |
| im getränkten Zustand | Maschinenrichtung | >4, vorzugsweise >60 |
| | Querrichtung | >10, vorzugsweise >20 |

Die Dehnfähigkeit vorteilhafter Tuches beträgt vorzugsweise

| | | |
|---|---|---|
| im trockenen Zustand | Maschinenrichtung | 15 % bis 100 %, bevorzugt 20 % und 50 % |
| | Querrichtung | 40 % bis 120 %, bevorzugt 50 % und 85 % |
| im getränkten Zustand | Maschinenrichtung | 15 % bis 100 %, bevorzugt 20 % und 40 % |
| | Querrichtung | 40 % bis 120 %, bevorzugt 50 % und 85 % |

Erfindungsgemäß ist die Verwendung von erfindungsgemäßen Zubereitungen zur Reinigung der Haut, insbesondere der Gesichtshaut.
Vorteilhaft ist insbesondere die Verwendung der erfindungsgemäßen Zubereitungen zur Reinigung des Augenlides sowie zur Entfernung von Make-up.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| **W/O-Reinigungsemulsionen** | | | |
|---|---|---|---|
| | **1** | **2** | **3** |
| Triglycerindiisostearat | 1,0 | 0,5 | 3,0 |
| Diglycerindipolyhydroxystearat | 1,0 | 1,5 | 2,0 |
| Paraffinöl | 12,5 | 10,0 | 7,5 |
| Vaseline | 1,0 | --- | --- |
| hydrierte Kokosglyceride | 0,5 | 0,1 | 0,25 |
| Decyloleat | 0,5 | 0,75 | 0,25 |
| Octyldodecanol | 0,5 | 1,0 | 0,25 |
| Aluminiumstearat | 0,04 | --- | 0,05 |
| Dicaprylylcarbonat | 0,1 | 0,05 | 1,0 |
| Magnesiumsulfat | 0,5 | 0,6 | 1,0 |
| Glycerin | --- | 5,0 | 5 |
| 2-Methyl-1,3-propandiol | 5,0 | 2,5 | 1,0 |
| Zitronensäure | 0,2 | 0,1 | 1,0 |
| Parfum | q,s, | q,s, | q,s, |
| Ethanol | 2,0 | --- | 10,0 |
| Capryl-/Caprinsäuretriglycerid | 2,0 | 2,5 | 0,5 |
| Methylparaben | 0,4 | 0,15 | 0,4 |
| Propylparaben | 0,3 | 0,4 | --- |
| lodopropynylbutylcarbamat | --- | --- | 0,1 |
| Wasser | ad 100 | ad 100 | ad 100 |

| **W/O- Reinigungsemulsionen (Referenzbeispiele)** | | | | | |
|---|---|---|---|---|---|
| | **6** | **7** | **8** | **9** | **10** |
| PEG-30 Dipolyhydroxystearat | --- | 0,5 | 0,25 | --- | 3,0 |
| Lanolin Alcohol | 1,0 | 1,5 | 1,75 | 3,0 | -- |
| Paraffinöl | 12,5 | 10,0 | 8,0 | 5,0 | 17,5 |
| Vaseline | 8,0 | 6,0 | 5,0 | 2,0 | 2,5 |
| 2-Methyl-1,3-propandiol | 5,0 | 2,5 | 15,0 | 7,5 | 10,0 |
| Hydriertes Polyisobuten | 0,5 | 0,75 | 1,0 | 2,0 | 0,25 |
| Octyldodecanol | 0,5 | 1,0 | 0,75 | 3,0 | 0,25 |
| Aluminiumstearat | 0,4 | 0,3 | 0,6 | 1,0 | 0,05 |
| Dicaprylylcarbonat | 0,1 | 0,05 | 0,15 | 0,5 | 1,0 |
| hydriertes Rizinusöl | 0,5 | 0,75 | 1,0 | 2,5 | 5,0 |
| Magnesiumsulfat | 0,5 | 0,6 | 0,5 | 0,7 | 1,0 |
| Glycerin | 3,0 | 5,0 | --- | --- | --- |
| Zitronensäure | 0,2 | 0,1 | 0,2 | 0,3 | 1,0 |
| Parfum | q,s, | q,s, | q,s, | q,s, | q,s, |
| 1,3 Butylenglykol | 2,0 | --- | 5,0 | --- | --- |
| Capryl-/Caprinsäuretriglycerid | 2,0 | 2,5 | 3,0 | 5,0 | 0,5 |
| Methylparaben | 0,4 | 0,15 | 0,05 | 0,3 | 0,4 |
| Propylparaben | 0,3 | 0,4 | 0,25 | 0,15 | --- |
| Wasser | ad 100 | ad 100 | ad 100 | ad 100 | ad 100 |

| **W/S-Reinigungsemulsionen (Referenzbeispiele)** | | | | | |
|---|---|---|---|---|---|
| | **11** | **12** | **13** | **14** | **15** |
| Cetyldimethiconecopolyol | 1,0 | --- | -- | 3,0 | 5,0 |
| Cylomethicon + PEG/PPG-18/18 Dimethicon | 10,0 | 12,5 | 25 | -- | -- |
| Cyclomethicon | 12,5 | 15 | 8 | 25,0 | 17,5 |
| Dimethicon | 5,0 | 13,0 | 5,0 | 12,0 | 15,0 |
| hydriertes Polyisobuten | 0,5 | 0,75 | 1,0 | 2,0 | 0,25 |
| Octyldodecanol | 0,5 | 1,0 | 0,75 | 3,0 | 0,25 |
| Panthenol | 0,5 | 1,0 | 0,75 | 0,25 | 0,1 |
| Natriumchlorid | 2,0 | 0,6 | 2,5 | 0,7 | 1,0 |
| Glycerin | 3,0 | -- | -- | -- | 15 |
| 2-Methyl-1,3-propandiol | 15,0 | 2,5 | 10,0 | 8,5 | 1,0 |
| Zitronensäure | 0,2 | 0,1 | 0,2 | 0,3 | 1,0 |
| Parfum | q,s, | q,s, | q,s, | q,s, | q,s, |
| Methylparaben | 0,4 | 0,1 | 0,05 | 0,3 | 0,4 |
| Propylparaben | 0,3 | 0,4 | 0,25 | 0,15 | -- |
| Cetyldimethicon | 0,5 | -- | 0,7 | -- | -- |
| lodopropynylbutylcarbamat | -- | -- | 0,05 | -- | 0,1 |
| modifizierte Stärke | -- | 2,5 | -- | 0,15 | -- |
| Wasser | ad 100 | ad 100 | ad 100 | ad 100 | ad 100 |

| **W/S- Reinigungsemulsionen (Referenzbeispiele)** | | | | | |
|---|---|---|---|---|---|
| | **16** | **17** | **18** | **19** | **20** |
| Cetyldimethiconecopolyol | 1,0 | -- | -- | 3,0 | 5,0 |
| Cylomethicon + PEG/PPG-18/18 Dimethicon | 10,0 | 12,5 | 25 | -- | -- |
| Cyclomethicon | 12,5 | 15 | 28,0 | 25,0 | 17,5 |
| Dimethicon | 5,0 | 13,0 | 5,0 | 12,0 | 15,0 |
| hydriertes Polyisobuten | 0,5 | 0,75 | 1,0 | 2,0 | 0,25 |
| Octyldodecanol | 0,5 | 1,0 | 0,75 | 3,0 | 0,25 |
| Panthenol | 0,5 | 1,0 | 0,75 | 0,25 | 0,1 |
| 2-Methyl-1,3-propandiol | 5,0 | 2,5 | 10,0 | 8,5 | 1,0 |
| Natriumchlorid | 2,0 | 0,6 | 2,5 | 0,7 | 1,0 |
| Sorbitol | 3,0 | 5,0 | 10,0 | 15,0 | 1,5 |
| Zitronensäure | 0,2 | 0,1 | 0,2 | 0,3 | 1,0 |
| Parfum | q,s, | q,s, | q,s, | q,s, | q,s, |
| Methylparaben | 0,4 | 0,1 | 0,05 | 0,3 | 0,4 |
| Propylparaben | 0,3 | 0,4 | 0,25 | 0,15 | -- |
| Stearyldimethicon | 0,5 | -- | 0,7 | -- | -- |
| lodopropynylbutylcarbamat | -- | -- | 0,05 | -- | 0,1 |
| modifizierte Stärke | -- | 2,5 | -- | 0,15 | -- |
| Wasser | ad 100 | ad 100 | ad 100 | ad 100 | ad 100 |

| **ÖI-in-Wasser Reinigungsemulsionen (Referenzbeispiele)** | | | | | |
|---|---|---|---|---|---|
| | **21** | **22** | **23** | **24** | **25** |
| Glycerylsterat | 1,0 | -- | -- | 3,0 | 5,0 |
| PEG-40-Stearat | 10,0 | -- | 5 | -- | -- |
| Triglycerinmethylglucosedistearat | -- | 5,5 | -- | -- | 2,5 |
| Sorbitanstearat | -- | 1,5 | 3 | -- | -- |
| Cyclomethicon | 1 | 2,5 | 5 | 7,5 | 3 |
| Dimethicon | 5,0 | 13,0 | 5,0 | 12,0 | 15,0 |
| Behenylalkohol | 0,1 | -- | 0,2 | 0,5 | -- |
| Stearylalkohol | -- | 1 | -- | 1 | -- |
| Cetylstearylalkohol | -- | -- | 0,1 | 1 | -- |
| hydriertes Polyisobuten | 0,5 | 0,75 | 1,0 | 2,0 | 0,25 |
| Octyldodecanol | 0,5 | 1,0 | 0,75 | 3,0 | 0,25 |
| 2-Methyl-1,3-propandiol | 5,0 | 2,5 | 10,0 | 8,5 | 1,0 |
| Panthenol | 0,5 | 1,0 | 0,75 | 0,25 | 0,1 |
| Glycerin | 3,0 | 5,0 | -- | -- | -- |
| Parfum | q,s, | q,s, | q,s, | q,s, | q,s, |
| Methylparaben | 0,4 | 0,1 | 0,05 | 0,3 | 0,4 |
| Propylparaben | 0,3 | 0,4 | 0,25 | 0,15 | -- |
| lodopropynylbutylcarbamat | -- | -- | 0,05 | -- | 0,1 |
| modifizierte Stärke | -- | 2,5 | -- | 0,15 | -- |
| Wasser | ad 100 | ad 100 | ad 100 | ad 100 | ad 100 |

| **Öl-in-Wasser- Reinigungsemulsionen (Referenzbeispiele)** | | | | |
|---|---|---|---|---|
| | **26** | **27** | **28** | **29** |
| Polyethylenglycol(21)stearylether | 1 | -- | 2,5 | 2 |
| Polyethylenglycol(2)stearylether | 1 | -- | 1,5 | 3 |
| Cetearylglucosid | -- | 8 | -- | -- |
| Cyclomethicon | 2,5 | 3 | 12,5 | 2 |
| Dimethicon | 5,0 | 13,0 | 5,0 | 12,0 |
| Behenylalkohol | 0,3 | 0,5 | -- | 0.1 |
| Stearylalkohol | 0,3 | -- | -- | 0,2 |
| Cetylstearylalkohol | 0,3 | 0,5 | -- | -- |
| hydriertes Polyisobuten | 0,5 | 0,75 | 1,0 | 2,0 |
| Octyldodecanol | 0,5 | 1,0 | 0,75 | 3,0 |
| 2-Methyl-1,3-propandiol | 15,0 | 2,5 | 10,0 | 8,5 |
| Panthenol | 0,5 | 1,0 | 0,75 | 0,25 |
| Glycerin | 3,0 | 5,0 | -- | -- |
| Parfum | q,s, | q,s, | q,s, | q,s, |
| Methylparaben | 0,4 | 0,1 | 0,05 | 0,3 |
| Propylparaben | 0,3 | 0,4 | 0,25 | 0,15 |
| Iodopropynylbutylcarbamat | -- | -- | 0,05 | -- |
| modifizierte Stärke | -- | 2,5 | -- | 0,15 |
| Wasser | ad 100 | ad 100 | ad 100 | ad 100 |

## Patentansprüche

1. Kosmetische Zubereitung zur Reinigung der Haut enthaltend
a) 1-8 Gewichts-% 2-Methyl-1,3-propandiol,
b) eine Ölkomponente gewählt aus den Verbindungen Octylpalmitat, Octylcocoat, Octylisostearat, Octyldodeceylmyristat, Cetearylisononanoat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat.

2. Tücher getränkt mit einer kosmetischen Zubereitung nach Anspruch 1.

3. Tücher nach Anspruch 2, **dadurch gekennzeichnet, dass** sie oberflächenstrukturiert sind.

4. Verwendung einer kosmetischen Zubereitung nach Anspruch 1 zur Reinigung der Haut, insbesondere der Gesichtshaut.

5. Verwendung einer kosmetischen Zubereitung nach Anspruch 1 zur Entfernung von Make-up.

## Claims

1. Cosmetic preparation for cleansing the skin comprising
a) 1-8% by weight of 2-methyl-1 ,3-propanediol,
b) an oil component selected from the compounds octyl palmitate, octyl cocoate, octyl isostearate, octyldodecyl myristate, cetearyl isononanoate, isopropyl palmitate, isopropyl stearate, isopropyl oleate, n-butyl stearate, n-hexyl laurate, n-decyl oleate, isooctyl stearate, isononyl stearate, isononyl isononanoate, 2-ethylhexyl palmitate, 2-hexyldecyl stearate, 2-octyldodecyl palmitate, stearyl heptanoate, oleyl oleate, oleyl erucate, erucyl oleate, erucyl erucate, tridecyl stearate and tridecyl trimellitate.

2. Wipes impregnated with a cosmetic preparation according to Claim 1.

3. Wipes according to Claim 2, **characterized in that** they are surface-structured.

4. Use of a cosmetic preparation according to Claim 1 for cleansing the skin, in particular the facial skin

5. Use of a cosmetic preparation according to Claim 1 for removing make-up.

## Revendications

1. Préparation cosmétique pour le nettoyage de la peau, comprenant
a) de 1 à 8 % en poids de 2-méthyl-1,3-propanediol,
b) un composant huileux choisi parmi les composés palmitate d'octyle, cocoate d'octyle, isostéarate d'octyle, myristate d'octyldodécyle, isononanoate de cétéaryle, palmitate d'isopropyle, stéarate d'isopropyle, oléate d'isopropyle, stéarate de n-butyle, laurate de n-hexyle, oléate de n-décyle, stéarate d'isooctyle, stéarate d'isononyle, isononanoate d'isononyle, palmitate de 2-éthylhexyle, stéarate de 2-hexyldécyle, palmitate de 2-octyldodécyle, heptanoate de stéaryle, oléate d'oléyle, érucate d'oléyle, oléate d'érucyle, érucate d'érucyle, stéarate de tridécyle et triméllitate de tridécyle.

2. Lingettes imprégnées d'une préparation cosmétique selon la revendication 1.

3. Lingettes selon la revendication 2, **caractérisées en ce qu'**elles sont structurées en surface.

4. Utilisation d'une préparation cosmétique selon la revendication 1 pour le nettoyage de la peau, en particulier de la peau du visage.

5. Utilisation d'une préparation cosmétique selon la revendication 1 pour l'élimination du maquillage.
